# EUROPEAN PATENT SPECIFICATION

(11) **EP 1 110 535 B1**
(45) Date of publication and mention of the grant of the patent: **21.02.2007**
(21) Application number: 00127243.4
(22) Date of filing: 15.12.2000
(51) Int. Cl.: A61K 8/02, A61K 8/29, A61K 8/19, A61Q 19/00

(54) **Cosmetic composition containing a hollow metal oxide plate powder**
Kosmetische Zusammensetzung enthaltend hohle plättchenförmige Metalloxid-Pulver
Composition cosmétique contenant des paillettes creuses d'oxydes métalliques

(30) Priority: 17.12.1999 JP 35979599; 18.01.2000 JP 2000009335
(43) Date of publication of application: 27.06.2001
(73) Proprietor: KAO CORPORATION, Tokyo 103-8210 (JP)
(72) Inventor: Nagatani, Noboru, Kao Corp. Research Lab., Tokyo 131-8501 (JP); Fukuda, Keiichi, Kao Corp. Research Lab., Tokyo 131-8501 (JP); Torizuka, Makoto, Kao Corp. Research Lab., Tokyo 131-8501 (JP); Igarashi, Takanori, Kao Corp. Research Lab., Tokyo 131-8501 (JP)
(74) Representative: HOFFMANN EITLE

(56) References cited:
- EP-A- 0 287 354
- EP-A- 0 768 277
- EP-A- 1 033 347
- US-A- 3 861 946
- US-A- 4 192 691
- US-A- 5 340 582
- US-A- 5 980 921
- PATENT ABSTRACTS OF JAPAN vol. 1997, no. 11, 28 November 1997 (1997-11-28) & JP 09 183709 A (NIPPON SHIKIZAI KOGYO KENKYUSHO:KK), 15 July 1997 (1997-07-15)
- PATENT ABSTRACTS OF JAPAN vol. 016, no. 443 (C-0985), 16 September 1992 (1992-09-16) & JP 04 154715 A (NOEVIR CO LTD;OTHERS: 01), 27 May 1992 (1992-05-27)

## Description

### BACKGROUND OF THE INVENTION

### Field of the Invention:

The present invention relates to a cosmetic composition according to claim 1 which can change the hue of the skin while giving the skin a feeling of transparence.

### Description of the Background Art:

In skin care cosmetic compositions and makeup cosmetic compositions comprising powder, it has heretofore been conducted with the object of changing the texture of the skin to incorporate powder having strong diffuse reflection so as to achieve a matte finish, or to incorporate powder having strong mirror reflection so as to achieve a lustrous finish.

In order to cover a dull looking skin (a state that the skin becomes dark and yellowish) caused by deteriorated blood circulation or aging, it has also been conducted to change the hue of the skin by incorporating a pigment of high covering effect, such as titanium oxide or iron oxide, or by adding redness of red iron oxide, lake pigment or organic pigment.

In order to cover a reddish face or acne spots on the other hand, it is conducted to change the hue of the skin by using a pigment of a green color that is complementary to red or using a pigment of a blue or violet color so as to give the skin a feeling of transparence.

When the pigment of high covering effect is used, however, a natural feeling tends to be lost. When the principle of complementary color is utilized, the chroma of the skin is lowered though the hue of the skin can be changed, resulting in a dull looking on the contrary. These problems are caused by the fact that the method depends on hue control based on subtractive color mixture, i.e., the more colors are overlaid, the lower the chroma of the appearance becomes, and so the color gets nearer to gray.

In order to solve these problems, it has been conducted in recent years to change the hue of the skin by using interference pearl powder composed mainly of mica coated with titanium oxide. When the conventional interference pearl powder is used, however, some change in tint is observed in a direction of regular reflection, but the chroma of the color is low, and so the hue of the skin has not been sufficiently changed as a whole.

As described above, the conventional cosmetic compositions have been insufficient to effectively change the hue of the skin while giving the skin a feeling of transparence.

US 3,861,946 relates to nacreous pigments comprising crystalline TiO₂ platelets, either supported on corresponding plate-like calcium sulphate anhydrite particles or self-supporting. The pigments are prepared by depositing amorphous TiO₂ coatings on plate-like gypsum substrate particles, calcining the resulting product to convert the gypsum to the anhydrite and crystallize the TiO₂, and, when an unsupported pigment is to be produced, dissolving out the anhydrite substrate. The crystalline TiO₂ is produced in the form of either rutile or anatase.

US 4,192,691 describes unsupported metal oxide plate nacreous pigments that are derived from metal oxide coated mica by a unique dissolution process and that show higher luster and coverage than the pigment from which they are derived.

EP 0 768 277 A1 relates to a process for producing zinc oxide fine particles which can be hollow. Possible uses thereof include use in a cosmetic composition.

US 5,340,582 discloses barium sulphate having a specific crystal structure and optical characteristics. The crystals have a plate-like structure of which the aspect ratio is 5 - 100 and the ratio of the square of the circumference of the plate and the area of the orthogonal projection plane is 20:1 - 150 :1. The cosmetic compositions into which said barium sulphate is incorporated show extendibility and adhesion to the skin and can hide spots or freckles on the skin.

### SUMMARY OF THE INVENTION

Accordingly, it is an object of the present invention to provide a cosmetic composition by which a change in the hue of the skin can be sufficiently recognized while giving the skin a feeling of transparence.

The present inventors have found that plate powder composed of a metal oxide and having a hollow therein is high in reflectance and produces surface interference light high in chroma, and that a cosmetic composition comprising said hollow plate-like powder can effectively change the hue of the skin while giving the skin a feeling of transparence.

According to the present invention, there is provided a cosmetic composition comprising metal oxide plate powder having a hollow structure, and titanium-dioxide coated mica.

### BRIEF DESCRIPTION OF THE DRAWINGS

Fig. 1 is an electron microscope photograph showing the appearance of metal oxide plate powder.
Fig. 2 is an electron microscope photograph showing the cross section of the metal oxide plate powder.

### DETAILED DESCRIPTION OF THE PREFERRED EMBODIMENTS

The term "plate powder" as used in the present invention means powder having an aspect ratio ranging from 5 to 100. The aspect ratio is particularly preferable within a range of 10 to 70 from the viewpoints of giving users a pleasant feeling upon use by lowering a coefficient of friction and making the production easy.

The average particle diameter of the metal oxide plate powder is preferably within a range of 12 µm or less, particularly 5 to 12 µm from the viewpoints of giving users a pleasant feeling upon use and lowering a feeling of glaringness.

Preferable examples of the metal oxide forming the plate powder include those having a high refractive index, such as titanium oxide, iron oxide, zirconium oxide and tin oxide. Among them, titanium oxide, particularly titanium oxide having a rutile type crystal structure is preferred. The metal oxide plate powder used in the present invention has a hollow inside.

The metal oxide plate powder having the hollow structure (hereinafter may be referred to as "the hollow plate powder (A)" in some cases) used in the present invention can be prepared in accordance with, for example, a process in which a platy powder base like mica or calcium sulfate is coated with a metal oxide, then the base substance is dissolved and removed by an acid and/or alkali to make the interior of the powder hollow (Japanese Patent Application Laid-Open No. 60565/1980 (US Patent No. 4,192,691), Japanese Patent Application Laid-Open No. 194757/1997 (US Patent No. 5,611,851) and Japanese Patent Application Laid-Open No. 45129/1974 (US Patent No. 3,861,946)). As the platy powder base, may be used mica, for example, muscovite, biotite or phlogopite or calcium sulfate, for example, gypsum dihydrate. Mica is particularly preferred. Examples of the acid used for dissolving the base include mineral acids such as hydrofluoric acid, hydrochloric acid, sulfuric acid, nitric acid and phosphoric acid. Examples of the alkali include sodium hydroxide and potassium hydroxide. Mica or calcium sulfate used as the base may remain to an extent of 20% by weight in the powder formed. However, the lower the content of the base material is, the better from the viewpoint of the intensity of interference color. In this respect, a method in which the plate powder coated with the metal oxide is brought into contact with an aqueous acid solution containing phosphoric acid or other mineral acid, and then into contact with an aqueous alkali solution is preferred because the content of the base material can be more lowered (Japanese Patent Application Laid-Open No. 194757/1997 (US Patent No. 5,611,851)). With respect to such hollow plate powder (A), the surface is not necessarily formed with a complete layer of the metal oxide, but a part thereof may be lacking.

Various color tones may be prepared in the resulting hollow plate powder (A) by controlling the optical thickness (coating thickness) of the metal oxide layer of the metal oxide-coated plate powder prior to the dissolution of the base material. For example, those powder which exhibit a blue to violet interference light do not become a dull color and can give a feeling of transparence toward the skin; those powder which exhibit a green interference light do also not become a dull color and can prevent the skin from looking red; and those powder which exhibit an orange to red interference light can make the skin look healthy and cover a dull looking skin.

Two or more kinds of the hollow plate powder (A) can be combined with each other, whereby various color tones may be prepared.

The surface of the hollow plate powder (A) used in the present invention may be subjected to a hydrophobicity-imparting treatment. The hydrophobicity-imparting treatment is conducted by treating the powder with a hydrophobicity-imparting agent in accordance with a method known *per se* in the art. Examples of the hydrophobicity-imparting agent include silicone oil, metal salts of fatty acids, alkyl phosphates, alkyl phosphate salts, N-mono-long chain aliphatic acylated basic amino acids and fluorine compounds having a perfluoroalkyl group.

Examples of the silicone oil include various kinds of linear silicones, cyclic silicones and modified silicones; examples of the metal salts of fatty acids include the calcium, magnesium, zinc and aluminum salts of fatty acids having 12 to 18 carbon atoms; examples of the alkyl phosphates and salts thereof include mono- or diesters having an alkyl or alkenyl group, which have 8 to 45 carbon atoms in total, and alkali metal salts or amine salts thereof; examples of the N-mono-long chain aliphatic acylated basic amino acids include basic amino acids with an acyl group having 8 to 22 carbon atoms, such as 2-ethylhexanoyl, capryloyl, caproyl, lauroyl, myristoyl, palmitoyl, stearoyl, isostearoyl, oleoyl, behenoyl, cocoyl, beef fatty acid acyl or hardened beef fatty acid acyl, bonded to an amino group of an α- or ω-position; and examples of the fluorine compounds having a perfluoroalkyl group include those described in US Patent No. 3,632,744, Japanese Patent Application Laid-Open No. 250074/1987, Japanese Patent Application Laid-Open No. 167209/1980, Japanese Patent Application Laid-Open No. 218603/1990.

The amount of the hydrophobicity-imparting agent for treating the hollow plate powder (A) is preferably 0.05 to 20% by weight, particularly 2 to 10% by weight from the viewpoints of imparting sufficient hydrophobicity and a good feel to the skin.

The cosmetic composition according to the present invention can be prepared in accordance with a method known *per se* in the art and provided as a skin care cosmetic composition such as toilet lotion, milky lotion or cream; or as a makeup cosmetic composition such as face powder, powdered foundation, oily foundation, creamy foundation, liquid foundation, concealer, lipstick, lip cream, cheek rouge, eye liner, eye shadow or eye brow.

The content of the hollow plate powder (A) in the cosmetic composition according to the present invention is preferably 0.1 to 30% by weight, particularly 0.5 to 30% by weight from the viewpoint of fully exhibiting the effect of the incorporation, while avoiding the unnaturalness due to a strong glossy feeling. In the case of the toilet lotion, milky lotion and cream, the hollow plate powder (A) is preferably contained in a proportion of 0.1 to 10% by weight, particularly 0.5 to 8% by weight based on the total weight of the composition. Besides, the hollow plate powder (A) is preferably incorporated in a proportion of 0.1 to 30% by weight, particularly 0.5 to 30% by weight in the case of the face powder; 0.1 to 30% by weight, particularly 0.5 to 30% by weight in the case of the powdered foundation and oily foundation; 0.1 to 30% by weight, particularly 0.5 to 25% by weight in the case of the creamy foundation, liquid foundation and concealer; 0.1 to 20% by weight, particularly 0.5 to 15% by weight in the case of the lipstick, lip cream; 0.1 to 30% by weight, particularly 0.5 to 25% by weight in the case of the cheek rouge and eye shadow; or 0.1 to 30% by weight, particularly 0.5 to 20% by weight in the case of the eye liner and eye brow.

Various kinds of powders are contained in the cosmetic composition according to the present invention, whereby various effects can be achieved in addition to the effect brought about by the hollow plate powder (A) that the hue of the skin can be changed while giving the skin a feeling of transparence.

A nacreous powder (component (E)), namely, titanium dioxide-coated mica is used together with the hollow plate powder (A), providing a cosmetic composition having no unnatural glaringness.

The titanium dioxide-coated mica of the component (E) is obtained by coating the surface of platy mica powder with titanium dioxide. The preferable average particle diameter of the component (E) is 12 µm or less, particularly a range of 5 to 12 µm. The average particle diameter is measured in accordance with the laser scattering particle size distribution analysis. The crystal form of titanium oxide is preferably a rutile type. However, other crystal forms may also be contained. Examples of commercially available products of such a component (E) include Timilon Supersilk MP1005 produced by Merck Co. and Flamenco SATIN Violet produced by Engelhard Corp.

The component (E) may also be used after subjected to a hydrophobicity-imparting treatment like the hollow plate powder (A). In the hydrophobicity-imparting treatment, the same hydrophobicity-imparting agent as that used in the hydrophobicity-imparting treatment of the hollow plate powder (A) is used.

The combined amount of the hollow plate powder (A) and the component (E) to be incorporated is preferably 0.1 to 50% by weight in order to suitably change the hue feeling to achieve a natural makeup finish free of an excess glossy feeling. A ratio (A)/(E) (weight ratio) is preferably within a range of 1/10 to 10/1, particularly 3/10 to 4/1 from the same point of view.

In the cosmetic composition according to the present invention, may be contained an oily substance (component (F)).

The oily substance preferably has a viscosity of 1,000 mPa·s or lower for the purpose of achieving a good feeling upon use without a sticky touch. When the viscosity is too low, however, the resulting cosmetic composition tends to give a creaky feel as a feeling upon use. Accordingly, preferred ones are those having a viscosity ranging from 0.1 to 1,000 mPa·s, particularly 2 to 500 mPa·s.

Examples of the component (F) include liquid paraffin, squalane, olive oil, ester oil, diglycerides, triglycerides, silicone oil, octyldodecyl myristate, neopentyl glycol dioctanate and fluorine-containing oily substances having a perfluoroalkyl group. Of these, liquid paraffin, squalane, ester oil, silicone oil and fluorine-containing oily substances having a perfluoroalkyl group are preferred.

The content of the component (F) in the cosmetic composition according to the present invention is preferably 0.5 to 60% by weight, particularly 2 to 50% by weight from the viewpoint of giving a pleasant feeling upon use without a sticky touch. With respect to the cosmetic composition according to claim 1 in which the hollow plate powder (A) is combined with the component (F), a ratio (A)/(F) (weight ratio) is preferably within a range of 1/10 to 100/1 from the viewpoints of improving spread upon coating and exhibiting the prescribed optical properties.

Other ingredients besides the components (A), (E) and (F), for example, surfactants, water-soluble polymers, other powders, moisturizers, preservatives, medicinally-effective ingredients, ultraviolet light absorbents, coloring matter, inorganic or organic salts, perfume bases, chelating agents, pH adjusters, water, may be incorporated into the cosmetic compositions according to the present invention as needed.

### Examples

### Preparation Example 1:

Mica (1.0 g) coated with anatase-type titanium oxide exhibiting green interference light was weighed out in a 250-mL polypropylene beaker, and distilled water (50 mL) was added thereto. A stirring rod coated with a plastic and a heating mantle were provided so as to control the temperature. Concentrated sulfuric acid (5.0 mL) was added from a glass pipette under stirring, and concentrated hydrofluoric acid (50%, 2.0 mL) was then added from a plastic pipette.

The resultant suspension was then heated at 70°C for 1 hour. After adding distilled water (200 mL), the resultant slurry was filtered through a Buchner funnel, and the filter cake was washed with distilled water (500 mL) to remove remaining acids. The thus-obtained powder was dried at 120°C for 30 minutes in an oven, thereby obtaining the intended metal oxide plate powder having a hollow inside.

### Preparation Example 2:

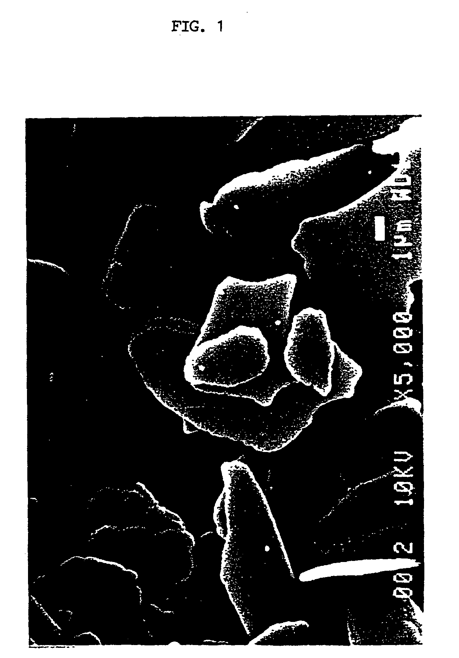

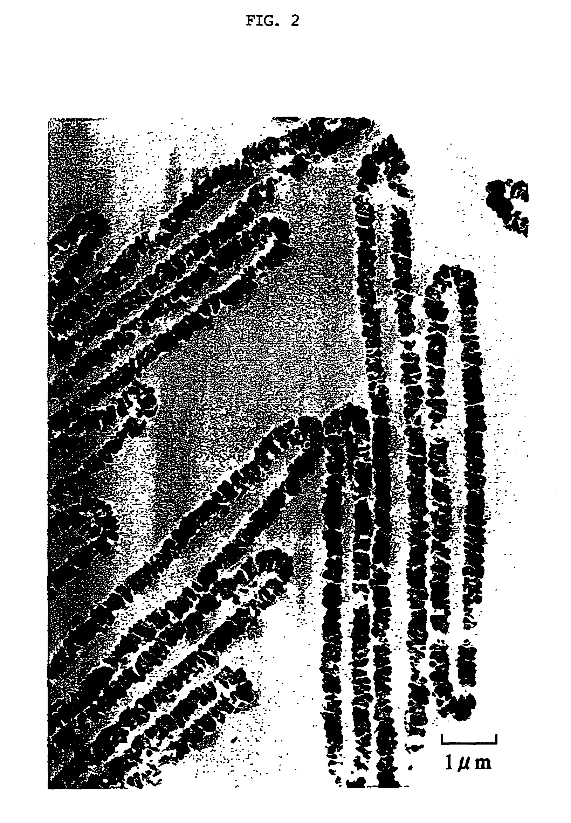

Mica (90 g) coated with rutile-type titanium oxide exhibiting blue interference light, sulfuric acid (124 g), phosphoric acid (60 g) and distilled water (300 mL) were mixed with one another to prepare a suspension. This suspension was refluxed at 120°C for 6 hours, cooled and then filtered. The resultant pressed cake was washed with distilled water until the pH of an eluate was 2, and distilled water (800 mL) was then added without drying the cake to prepare a slurry. Sodium hydroxide (83 g) was added to prepare a 9% sodium hydroxide solution. The solution was heated to 60°C. After stirring for 1 hour, the solution was filtered, and the resultant pressed cake was washed until the pH of an eluate was 10. The formed product was dried at 100°C for 14 hours to obtain the intended metal oxide plate powder. The metal oxide plate powder thus obtained had a hollow structure. The electron microscope photographs thereof are shown in Figs. 1 and 2.

### Reference Example 1 and Comparative Examples 1 and 2: (Powdered foundation)

### (Preparation process)

Components (1) to (9) shown in Table 1 are mixed and ground. The ground product is transferred to a high-speed blender, and components (10) to (12) mixed together at 80°C are added to the ground product, followed by uniform mixing. After a component (13) is added to the resultant mixture to mix them, the resultant mixture is ground again and passed through a sieve. This product was compression-molded in a metal tray.

### (Evaluation method)

Each powdered foundation was evaluated with respect to no dullness, brightness, natural feeling and transparence when applying it to the skin. The evaluation was conducted by 20 expert panelists to give 5 points to "Good", 4 points to "Somewhat good", 3 points to "Fair", 2 points to "Somewhat poor" or 1 point to "Poor" as to each item, thereby calculating out an average value of the 20 panelists. The results are shown in Table 1.

**Table 1**

| | | | Reference Ex. 1 | Comp. Ex. 1 | Comp. Ex. 2 |
|---|---|---|---|---|---|
| (1) | Mica | | Balance | Balance | Balance |
| (2) | Nylon powder | | 10 | 10 | 10 |
| (3) | Powder of Preparation Ex. 2 | | 10 | - | - |
| (4) | Raw powder of Preparation Ex. 2 | | - | - | 10 |
| (5) | Talc | | 20 | 20 | 20 |
| (6) | Titanium oxide | | 10 | 10 | 10 |
| (7) | Red iron oxide | | 1.8 | 1.8 | 1.8 |
| (8) | Yellow iron oxide | | 2.5 | 2.5 | 2.5 |
| (9) | Black iron oxide | | 0.1 | 0.1 | 0.1 |
| (10) | Liquid paraffin | | 8 | 8 | 8 |
| (11) | Bees wax | | 2 | 2 | 2 |
| (12) | Preservative | | q.s. | q.s. | q.s. |
| (13) | Perfume base | | Trace | Trace | Trace |
| Total | | | 100 | 100 | 100 |
| Evaluation | | No dullness | 4.5 | 2.3 | 3.5 |
| | | Brightness | 4.3 | 2.5 | 4.0 |
| | | Natural feeling | 4.6 | 2.5 | 1.5 |
| | | Transparence | 4.8 | 1.5 | 3.0 |

As apparent from Table 1, the cosmetic composition according to the present invention comprising the hollow plate powder (A) can naturally change the tint of the skin, gives no dull appearance, achieves a finish bright and high feeling of transparence; showing remarkably high effects compared with the cosmetic composition (Comparative Example 2) comprising ordinary titanium oxide-coated mica.

### Reference Example 2 and Comparative Examples 3 and 4: (Powdered foundation)

Powdered foundations having their corresponding compositions shown in Table 2 were prepared in accordance with the method as described in reference Example 1 to evaluate them as to finish. The results are shown in Table 3.

**Table 2**

| (%) | | | | |
|---|---|---|---|---|
| Component | | Reference Ex. 2 | Comp. Ex. 3 | Comp. Ex. 4 |
| (1) | Mica | Balance | Balance | Balance |
| (2) | Nylon powder | 10 | 10 | 10 |
| (3) | Powder of Preparation Ex. 2 | 5 | - | - |
| (4) | Rutile-type titanium oxide-coated mica | - | 5 | 5 |
| (5) | Talc | 20 | 20 | 20 |
| (6) | Platy barium sulfate ^{*1} | 10 | 10 | - |
| (7) | Barium sulfate ^{*2} | - | - | 10 |
| (8) | Titanium oxide | 10 | 10 | 10 |
| (9) | Red iron oxide | 0.8 | 0.8 | 0.8 |
| (10) | Yellow iron oxide | 2.5 | 2.5 | 2.5 |
| (11) | Black iron oxide | 0.1 | 0.1 | 0.1 |
| (12) | Liquid paraffin | 8 | 8 | 8 |
| (13) | Bees wax | 2 | 2 | 2 |
| (14) | Preservative | q.s. | q.s. | q.s. |
| (15) | Perfume base | Trace | Trace | Trace |

| | | | | |
|---|---|---|---|---|
| *1 Refractive index: 1.65, diffuse transmittance: 73%, total transmittance: 88%, aspect ratio: 58, R = 88:1, butterfly form, *2 Refractive index: 1.76, diffuse transmittance: 68%, total transmittance: 80%, aspect ratio: 1.5, R = 78:1, plate form. | | | | |

### (Evaluation method)

Each powdered foundation was evaluated with respect to no dullness, brightness, natural feeling, transparence and hiding of spots and freckles when applying it to the skin. The evaluation was conducted by 14 expert panelists to give 5 points to "Good", 4 points to "Somewhat good", 3 points to "Fair", 2 points to "Somewhat poor" or 1 point to "Poor" as to each item, thereby calculating out an average value of the 14 panelists.

**Table 3**

| Evaluation item | Ex. 2 | Comp. Ex. 3 | Comp. Ex. 4 |
|---|---|---|---|
| No dullness | 4.4 | 1.5 | 1.5 |
| Brightness | 4.7 | 1.6 | 1.8 |
| Natural feeling | 4.8 | 1.3 | 1.5 |
| Transparence | 4.0 | 2.5 | 2.5 |
| Hiding of spots and freckles | 4.5 | 3.5 | 1.8 |

### Reference Example 3 and Comparative Example 5: (Powdered foundation)

Powdered foundations having their corresponding compositions shown in Table 4 were prepared in accordance with the method shown in reference Example 1 to evaluate them as to finish and retention of makeup in accordance with the following evaluation method. The results are shown in Table 5.

**Table 4**

| (%) | | | |
|---|---|---|---|
| Component | | Reference Ex. 3 | Comp. Ex. 5 |
| (1) | Mica | Balance | Balance |
| (2) | Nylon powder | 10 | 10 |
| (3) | Powder of Preparation Ex. 2 | 5 | - |
| (4) | Rutile-type titanium oxide-coated mica | - | 5 |
| (5) | Talc | 20 | 20 |
| (6) | Zinc oxide (specific surface area: 50 m²/g, FINEX-50, product of Sakai Chemical Industry Co., Ltd.) | 4 | 4 |
| (7) | Titanium oxide | 10 | 10 |
| (8) | Red iron oxide | 0.8 | 0.8 |
| (9) | Yellow iron oxide | 2.5 | 2.5 |
| (10) | Black iron oxide | 0.1 | 0.1 |
| (11) | Liquid paraffin | 8 | 8 |
| (12) | Bees wax | 2 | 2 |
| (13) | Preservative | q.s. | q.s. |
| (14) | Perfume base | Trace | Trace |

### (Evaluation method)

Each powdered foundation was evaluated with respect to no dullness, brightness, natural feeling, transparence and retention of makeup upon elapsed time of 4 hours after application (under ordinary living environment in a room). The evaluation was conducted by 14 expert panelists to give 5 points to "Good", 4 points to "Somewhat good", 3 points to "Fair", 2 points to "Somewhat poor" or 1 point to "Poor" as to each item, thereby calculating out an average value of the 14 panelists.

**Table 5**

| Evaluation item | | Reference Ex. 2 | Comp. Ex. 4 |
|---|---|---|---|
| Right after application | No dullness | 4.5 | 1.5 |
| | Brightness | 4.7 | 1.6 |
| | Natural feeling | 4.8 | 1.3 |
| | Transparence | 4.7 | 0.9 |
| After 4 hours | No dullness | 4.1 | 1.0 |
| | Brightness | 4.5 | 1.2 |
| | Natural feeling | 4.1 | 1.0 |
| | Transparence | 4.2 | 0.5 |
| | Retention of makeup | 4.5 | 4.2 |

### Reference Example 4 and Comparative Example 6: (Powdered foundation)

Powdered foundations having their corresponding compositions shown in Table 6 were prepared in accordance with the method as described in Reference Example 1 to evaluate them as to makeup performance properties in accordance with the following evaluation method. The results are shown in Table 7.

**Table 6**

| (%) | | | |
|---|---|---|---|
| Component | | Reference Ex. 4 | Comp. Ex. 6 |
| (1) | Mica | Balance | Balance |
| (2) | Nylon powder | 10 | 10 |
| (3) | Powder of Preparation Ex. 2 | 10 | - |
| (4) | Monodisperse spherical silica ^{*3} | 10 | 10 |
| (5) | Talc | 20 | 20 |
| (6) | Titanium oxide | 10 | 10 |
| (7) | Red iron oxide | 1.8 | 1.8 |
| (8) | Yellow iron oxide | 2.5 | 2.5 |
| (9) | Black iron oxide | 0.1 | 0.1 |
| (10) | Liquid paraffin | 8 | 8 |
| (11) | Bees wax | 2 | 2 |
| (12) | Preservative | q.s. | q.s. |
| (13) | Perfume base | Trace | Trace |

| | | | |
|---|---|---|---|
| *3: Average particle diameter: 0.4 µm, coefficient of variation: 4%, refractive index: 1.45. | | | |

### (Evaluation method)

Each powdered foundation was evaluated with respect to no dullness, brightness, natural feeling, transparence, no creaky feeling, no powder-like feeling, adhesion to the skin, spreadability on the skin and texture of finish when applying it to the skin. The evaluation was conducted by 20 expert panelists to give 5 points to "Good", 4 points to "Somewhat good", 3 points to "Fair", 2 points to "Somewhat poor" or 1 point to "Poor" as to each item, thereby calculating out an average value of the 20 panelists.

**Table 7**

| Evaluation item | Reference Ex. 4 | Comp. Ex. 6 |
|---|---|---|
| No dullness | 4.5 | 2.0 |
| Brightness | 4.3 | 2.0 |
| Natural feeling | 4.5 | 2.0 |
| Transparence | 4.8 | 2.2 |
| No creaky feel | 4.5 | 3.0 |
| No powder-like feeling | 4.8 | 2.5 |
| Adhesion | 4.3 | 2.0 |
| Spreadability | 4.6 | 3.2 |
| Texture of finish | 4.8 | 3.5 |

Examples 5 to 7 and Comparative Examples 7 and 8:

### (Powdered foundation)

Powdered foundations having their corresponding compositions shown in Table 8 were prepared in accordance with the method as described in Example 1 to evaluate them as to finish in accordance with the following evaluation method. The results are shown in Table 9.

**Table 8**

| (%) | | | | | | |
|---|---|---|---|---|---|---|
| Component | | Ex. 5 | Comp. Ex. 7 | Ex. 6 | Ex. 7 | Comp. Ex. 8 |
| (1) | Mica | Bal. | Bal. | Bal. | Bal. | Bal. |
| (2) | Nylon powder | 10 | 10 | 10 | 10 | 10 |
| (3) | Powder of Preparation Ex. 2 | 8 | - | 1 | 11 | - |
| (4) | Talc | 20 | 20 | 20 | 20 | 20 |
| (5) | Titanium oxide-coated mica ^{*4} | 4 | 12 | 11 | 1 | - |
| | (blue interference light system) | | | | | |
| (6) | Titanium oxide | 10 | 10 | 10 | 10 | 10 |
| (7) | Red iron oxide | 0.8 | 0.8 | 0.8 | 0.8 | 0.8 |
| (8) | Yellow iron oxide | 2.3 | 2.3 | 2.3 | 2.3 | 2.3 |
| (9) | Black iron oxide | 0.2 | 0.2 | 0.2 | 0.2 | 0.2 |
| (10) | Liquid paraffin | 8 | 8 | 8 | 8 | 8 |
| (11) | Bees wax | 2 | 2 | 2 | 2 | 2 |
| (12) | Preservative | q.s. | q.s. | q.s. | q.s. | q.s. |
| (13) | Perfume base | Trace | Trace | Trace | Trace | Trace |

| | | | | | | |
|---|---|---|---|---|---|---|
| *1 Flamenco SATIN Blue (rutile type), product of Engelhard Corp. | | | | | | |

### (Evaluation method)

Each powdered foundation was evaluated with respect to no dullness, brightness, natural feeling, transparence and no feeling of glaringness when applying it to the skin. The evaluation was conducted by 14 expert panelists to give 5 points to "Good", 4 points to "Somewhat good", 3 points to "Fair", 2 points to "Somewhat poor" or 1 point to "Poor" as to each item, thereby calculating out an average value of the 14 panelists.

**Table 9**

| Evaluation item | Ex. 5 | Comp. Ex. 7 | Ex. 6 | Ex. 7 | Comp. Ex. 8 |
|---|---|---|---|---|---|
| No dullness | 4.5 | 3.3 | 4.4 | 4.5 | 1.0 |
| Brightness | 4.5 | 2.5 | 3.3 | 3.5 | 1.0 |
| Transparence | 4.7 | 2.9 | 4.5 | 4.6 | 1.3 |
| No feeling of glaringness | 4.3 | 2.5 | 3.6 | 3.6 | 2.1 |

As apparent from the results shown in Table 9, when the cosmetic compositions according to the present invention comprising the hollow plate powder (A) and the component (E) were separately used, in each case, a high feeling of transparence was given, and a good finish free of unnaturalness was achieved.

### Reference Example 8: (Powdered foundation)

A powdered foundation having a composition shown in Table 10 was prepared in accordance with the method as described in Reference Example 1 to evaluate it as to finish and retention of makeup in accordance with the following evaluation method. The results are shown in Table 11.

**Table 10**

| (%) | | |
|---|---|---|
| Component | | Reference Ex. 4 |
| (1) | Mica | Balance |
| (2) | Nylon powder | 10 |
| (3) | Powder of Preparation Ex. 2 | 5 |
| (4) | Talc | 20 |
| (5) | Zinc oxide | 4 |
| (6) | Titanium oxide | 10 |
| (7) | Red iron oxide | 0.8 |
| (8) | Yellow iron oxide | 2.5 |
| (9) | Black iron oxide | 0.1 |
| (10) | Dimethyl polysiloxane (viscosity: 10 mPa·s) | 8 |
| (11) | Bees wax | 2 |
| (12) | Preservative | q.s. |
| (13) | Perfume base | Trace |

### (Evaluation method)

The powdered foundation was evaluated with respect to no dullness, brightness, natural feeling, transparence and spreadability when applying it to the skin. The evaluation was conducted by 14 expert panelists to give 5 points to "Good", 4 points to "Somewhat good", 3 points to "Fair", 2 points to "Somewhat poor" or 1 point to "Poor" as to each item, thereby calculating out an average value of the 14 panelists.

**Table 11**

| Evaluation item | Reference Ex. 8 |
|---|---|
| No dullness | 4.5 |
| Brightness | 4.7 |
| Natural feeling | 4.8 |
| Transparence | 4.7 |
| Spreadability | 4.5 |

### Reference Example 9: (Creamy foundation)

| (Composition) | | (%) |
|---|---|---|
| (1) | Stearic acid | 5.5 |
| (2) | Lipophilic glyceryl monostearate | 2.5 |
| (3) | Cetostearyl alcohol | 1.0 |
| (4) | Monolaurylpropylene glycol | 3.0 |
| (5) | Squalane | 7.0 |
| (6) | Olive oil | 8.0 |
| (7) | Purified water | Balance |
| (8) | Preservative | q.s. |
| (9) | Triethanolamine | 1.2 |
| (10) | Sorbit | 3.0 |
| (11) | Titanium dioxide | 8.0 |
| (12) | Talc | 5.0 |
| (13) | Color pigments (black iron oxide, red iron oxide, yellow iron oxide) | q.s. |
| (14) | Platy barium sulfate ^{*5} | 4.0 |
| (15) | Powder of Preparation Example 1 | 5.0 |
| (16) | Perfume base | Trace |

| | | |
|---|---|---|
| *5: Refractive index: 1.63, diffuse transmittance: 75%, total transmittance: 89%. | | |

### Reference Example 10 : (Face powder)

| (Composition) | | (%) |
|---|---|---|
| (1) | Mica | Balance |
| (2) | Metal oxide plate powder of hollow structure ^{*6} | 8.0 |
| (3) | Platy barium sulfate ^{*7} | 30.0 |
| (4) | Talc | 30.0 |
| (5) | Titanium dioxide | 0.5 |
| (6) | Red iron oxide | 0.1 |
| (7) | Yellow iron oxide | 0.1 |
| (8) | Black iron oxide | 0.01 |
| (9) | Magnesium stearate | 10.0 |
| (10) | Preservative | q.s. |

| | | |
|---|---|---|
| *6: Powder obtained by using, as a raw powder, mica coated with rutile-type titanium oxide exhibiting violet interference light in Preparation Example 2, *5: Refractive index: 1.63, diffuse transmittance: 71%, total transmittance: 86%. | | |

### Reference Example 11: (Solid face powder)

| (Composition) | | (%) |
|---|---|---|
| (1) | Mica | Balance |
| (2) | Metal oxide plate powder of hollow structure ^{*8} | 25.0 |
| (3) | Platy barium sulfate ^{*9} | 6.0 |
| (4) | Talc | 20.0 |
| (5) | Titanium dioxide | 0.5 |
| (6) | Red iron oxide | 0.1 |
| (7) | Yellow iron oxide | 0.1 |
| (8) | Black iron oxide | 0.01 |
| (9) | Liquid paraffin | 8.0 |
| (10) | Bees wax | 2.0 |
| (11) | Preservative | q.s. |
| (12) | Perfume base | Trace |

| | | |
|---|---|---|
| *8: Powder obtained by treating powder (1 part by weight) obtained by using, as a raw material, mica coated with rutile-type titanium oxide exhibiting violet interference light in Preparation Example 2 with dimethyl polysiloxane (0.02 parts by weight), *9: Refractive index: 1.64, diffuse transmittance: 72%, total transmittance: 88%. | | |

### Reference Example 12: (Cheek rouge)

| | | |
|---|---|---|
| (Composition) | | (%) |
| (1) | Mica | Balance |
| (2) | Metal oxide plate powder of hollow | 20.0 |
| | structure ^{*10} | |
| (3) | Talc | 20.0 |
| (4) | Platy barium sulfate (the same as that used in Example 2) | 7.0 |
| (5) | Titanium dioxide | 4.0 |
| (6) | Zinc stearate | 5.0 |
| (7) | Rice starch | 5.0 |
| (8) | Coloring matter | 3.0 |
| (9) | Liquid paraffin | 3.0 |
| (10) | Preservative | q.s. |
| (11) | Perfume base | Trace |

| | | |
|---|---|---|
| *10: Powder obtained by treating powder (1 part by weight) prepared by changing the raw powder in Preparation Example 1 to mica coated with anatase-type titanium oxide exhibiting red interference light with lauroyllysine (0.05 parts by weight). | | |

### Reference Example 13 : (Eye shadow)

| (Composition) | | (%) |
|---|---|---|
| (1) | Mica | Balance |
| (2) | Metal oxide plate powder of hollow structure ^{*11} | 20.0 |
| (3) | Talc | 5.0 |
| (4) | Platy barium sulfate (the same as that used in Reference Example 3) | 7.0 |
| (5) | Mica titanium | 5.0 |
| (6) | Zinc stearate | 5.0 |
| (7) | Zinc laurate | 3.0 |
| (8) | Color pigments (black iron oxide, red iron oxide, yellow iron oxide) | 10.0 |
| (9) | Liquid paraffin | 7.0 |
| (10) | Preservative | q.s. |
| (11) | Perfume base | Trace |

| | | |
|---|---|---|
| *11: Powder obtained by using, as a raw powder, mica coated with rutile-type titanium oxide exhibiting red interference light in Preparation Example 2. | | |

### Reference Example 14 : (Liquid foundation)

| (Composition) | | (%) |
|---|---|---|
| (1) | Pigments subjected to hydrophobicity-imparting treatment (treated with methyl hydrogen polysiloxane) Titanium dioxide | 6.0 |
| | Iron oxides (red, yellow and black) | q.s. |
| (2) | Polymethylsilsesquioxane powder 4.0 ("Tospearl 145", product of Toshiba Silicone Co., Ltd.) | |
| (3) | Metal oxide plate powder of hollow structure ^{*12} | 5.0 |
| (4) | Platy barium sulfate (obtained by treating that (1 part by weight) used in Example 9 with dimethyl polysiloxane (0.02 parts by weight) | 4.0 |
| (5) | Octamethylcyclotetrasiloxane | 20.0 |
| (6) | Dimethyl polysiloxane ("Silicone KF-96A", product of Shin-Etsu Chemical Co., Ltd.) | 10.0 |
| (7) | Dimethyl polysiloxane·polyoxyalkylene copolymer ("SH3775C", product of Toray Dow Corning.Co., Ltd.) | 1.0 |
| (8) | Glycerol | 2.0 |
| (9) | Purified water | Balance |

| | | |
|---|---|---|
| *12: Powder obtained by treating the powder (1 part by weight) of Preparation Example 2 with methyl hydrogen polysiloxane (0.02 parts by weight). | | |

### Reference Example 15 : (Eye liner)

| (Composition) | | (%) |
|---|---|---|
| (1) | Carnauba wax | 5.0 |
| (2) | Bees wax | 1.0 |
| (3) | Miorocrystalline wax | 10.0 |
| (4) | White vaseline | 1.0 |
| (5) | Organic bentonite | 0.5 |
| (6) | Light liquid paraffin | Balance |
| (7) | Powder obtained by treating powder (1 part by weight) of Preparation Example 1 with dimethyl polysiloxane (0.02 parts by weight) | 10.0 |
| (8) | Platy barium sulfate (that obtained by treating that (1 part by weight) used in Example 12 with dimethyl polysiloxane (0.02 parts by weight) | 5.0 |
| (9) | Titanium dioxide | 3.0 |
| (10) | Carbon black | 2.0 |
| (11) | Preservative | q.s. |

### Reference Example 16 : (O/W type cream)

| (Composition) | | (%) |
|---|---|---|
| (1) | Bees wax | 5.5 |
| (2) | Cetanol | 4.5 |
| (3) | Hydrogenated lanolin | 7.0 |
| (4) | Squalane | 33.0 |
| (5) | Glycerol fatty acid | 3.5 |
| (6) | Lipophilic glyceryl monostearate | 2.0 |
| (7) | Polyoxyethylene (20 E.O.) sorbitan monolaurate | 2.0 |
| (8) | Powder of Preparation Example 1 | 4.0 |
| (9) | Platy barium sulfate (the same as that used in Example 1) | 4.0 |
| (10) | Perfume base | 0.1 |
| (11) | Preservative | 0.2 |
| (12) | Antioxidant | 0.1 |
| (13) | Propylene glycol | 10.0 |
| (14) | Purified water | Balance |

As described above, the cosmetic compositions according to the present invention can give the skin a high feeling of transparence, produce a natural finish and change the hue of the skin.

(Examples denoted as Reference Examples are not within the scope of the present invention).

## Claims

1. A cosmetic composition comprising (A) metal oxide plate powder having a hollow structure and (E) titanium dioxide-coated mica.

2. The cosmetic composition of claim 1, wherein the ratio by weight of component (A) to component (E) is in the range of 1/10 to 10/1.

## Patentansprüche

1. Kosmetische Zusammensetzung, umfassend (A) plättchenförmiges Metalloxidpulver, das eine hohle Struktur aufweist, und (E) Titandioxid-beschichteten Glimmer:

2. Kosmetische Zusammensetzung gemäss Anspruch 1, worin das Gewichtsverhältnis der Komponente (A) zur Komponente (E) im Bereich von 1:10 bis 10:1 ist.

## Revendications

1. Composition cosmétique comprenant (A) des paillettes d'oxyde de métal ayant une structure creuse et (E) un mica revêtu d'oxyde de titane.

2. Composition cosmétique selon la revendication 1, dans laquelle le rapport en poids du composant (A) sur le composant (E) est dans la gamme de 1/10 à 10/1.
